# EUROPEAN PATENT SPECIFICATION

(11) **EP 4 034 181 B1**
(45) Date of publication and mention of the grant of the patent: **13.03.2024**
(21) Application number: 20786397.8
(22) Date of filing: 22.09.2020
(51) Int. Cl.: A61L 29/08, A61L 29/12, A61L 29/14, A61L 29/16

(54) **REUSABLE URINARY CATHETER PRODUCTS**
WIEDERVERWENDBARE HARNKATHETERPRODUKTE
PRODUITS DE CATHÉTER URINAIRE RÉUTILISABLES

(30) Priority: 24.09.2019 US 201962905056 P
(43) Date of publication of application: 03.08.2022
(60) Divisional application: 24154693.6
(73) Proprietor: Hollister Incorporated, Libertyville, IL 60048 (US)
(72) Inventor: FARRELL, David, J., Libertyville, IL 60048 (US); O'FLYNN, Padraig, M., Libertyville, IL 60048 (US); CLARKE, John, T., Libertyville, IL 60048 (US)
(74) Representative: FRKelly
(86) International application number: PCT/US2020/052051
(87) International publication number: WO 2021/061674

(56) References cited:
- WO-A1-2012/177803
- YUKI SEKIGUCHI ET AL: "Self-sterilizing catheters with titanium dioxide photocatalyst thin films for clean intermittent catheterization: Basis and study of clinical use", INTERNATIONAL JOURNAL OF UROLOGY, vol. 14, no. 5, 1 May 2007 (2007-05-01), pages 426-430, XP055043776, ISSN: 0919-8172, DOI: 10.1111/j.1442-2042.2007.01743.x
- IRAM B DITTA ET AL: "Photocatalytic antimicrobial activity of thin surface films of TiO2, CuO and TiO2/CuO dual layers on Escherichia coli and bacteriophage T4", APPLIED MICROBIOLOGY AND BIOTECHNOLOGY, SPRINGER, BERLIN, DE, vol. 79, no. 1, 4 March 2008 (2008-03-04), pages 127-133, XP019623564, ISSN: 1432-0614
- ARJUNAN SUGANYA ET AL: "Study on plasma pre-functionalized PVC film grafted with TiO/PVP to improve blood compatible and antibacterial properties", JOURNAL OF PHYSICS D: APPLIED PHYSICS, INSTITUTE OF PHYSICS PUBLISHING LTD, GB, vol. 50, no. 14, 9 March 2017 (2017-03-09) , page 145402, XP020315245, ISSN: 0022-3727, DOI: 10.1088/1361-6463/AA5F06 [retrieved on 2017-03-09]

## Description

The present application claims the benefit of and priority to U.S. Provisional Application No. 62/905,056, filed September 24, 2019.

### Field of the Disclosure

The present disclosure generally relates to urinary catheters. More particularly, the present disclosure relates to reusable urinary catheter products.

### Background

Catheters are used to treat many different types of medical conditions and typically include an elongated shaft that is inserted into and through a passageway or lumen of the body. Catheters, and in particular intermittent catheters, are commonly used by those who suffer from various abnormalities of the urinary system, such as urinary incontinence. With the advent of intermittent catheters, individuals with urinary system abnormalities can self-insert and selfremove intermittent catheters several times a day.

Urinary catheters are frequently provided as disposable, single-use items. A user will remove the catheter from a package, use the catheter once, and then dispose of the catheter and the package. Reusable urinary catheters could, thus, be advantageous in reducing the amount of waste created by the use of disposable catheters, but there are various challenges associated with the use of reusable catheters (including storage, transport, and sterilization) that must be overcome before widespread acceptance and use of reusable catheters.

There is a need for reusable catheter products and methods of sterilizing the same.

WO 2012/177803 A1 relates to a method and system for inhibiting a biofilm on the surface of an implanted catheter such as an urinary catheter for killing microorganisms in a biofilm that is present or forming on the catheter surface, and/or preventing biofilm growth. The implantable catheter can be photocatalycally disinfected. It comprises an elongated catheter body having an outer photocatalytic layer on the outer surface that undergoes a photocatalysis reaction upon irradiation with UV to generate reactive oxygen species. The photocatalytic layer is composed of crystalline titanium dioxide.

### Summary

The scope of protection is defined by the claims.

The present invention relates to a reusable urinary catheter product according to claims 1-13.

### Brief Description of the Drawings

Fig. 1 is perspective view of a reusable catheter in accordance with the present disclosure;
Fig. 2 is a partial enlarged cross-sectional view of one embodiment of a catheter shaft of Fig. 1;
Fig. 3 is a partial enlarged cross-sectional view of another embodiment of a catheter shaft of Fig. 1;
Fig. 4 is a schematic illustration of a catheter product including a light source; and
Fig. 5 is a front elevational view of another embodiment of a reusable catheter product including a case.

### Description

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

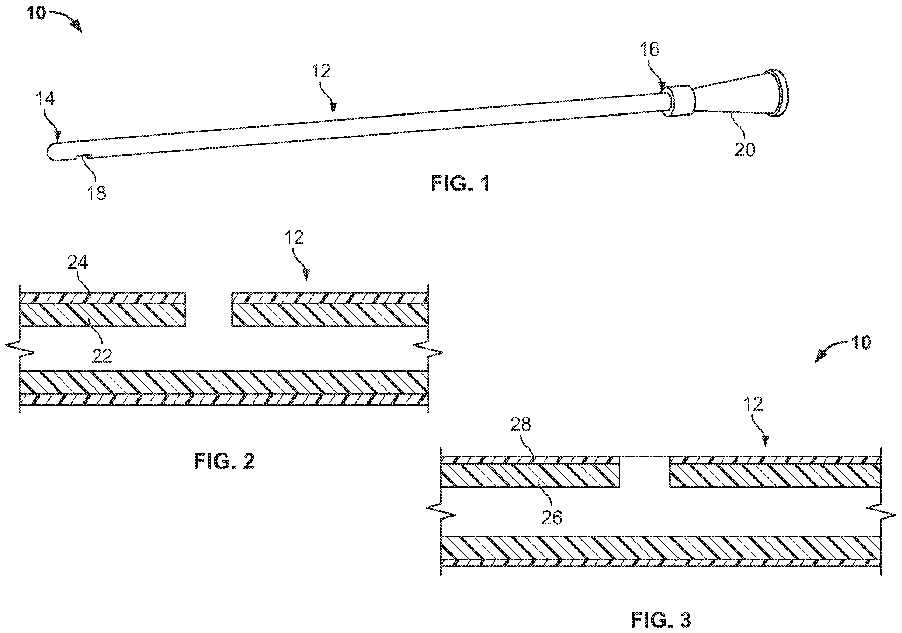

Reusable urinary catheter products according to the present disclosure and their individual components may be variously configured without departing from the scope of the claimed invention, but the reusable urinary catheter as shown in Fig.1 is not according to the claimed invention.

Fig. 1 illustrates one embodiment of a reusable intermittent catheter 10 of the present disclosure. The intermittent catheter 10 may be used, disinfected and then reused. The reusable catheter 10 includes a catheter shaft 12. The catheter shaft 12 includes a proximal insertion end 14 and a distal end 16. The proximal insertion end 14 includes a drainage hole or eyelet 18 for the drainage of urine from the bladder and into the catheter shaft 12. A drainage member 20, such as a funnel, is located at the distal end 16 of the catheter shaft 12.

The catheter shaft 12 is made from polymer(s) and includes photoactive titanium dioxide. Furthermore, the drainage member 20 also may be made from polymer(s) and include a photoactive titanium dioxide. The polymer(s) of the shaft and the drainage member may be the same or different polymers. Such polymers may include thermoplastic polymers, such as polyvinyl chloride, thermoplastic olefins, such as polypropylene, polyethylene, block copolymer polypropylene, and/or thermoplastic elastomers, such as Styrenic block copolymers, polyurethanes etc. The polymers may include biodegradable polymers and biodegradable thermoplastic polymers.

Photoactive titanium dioxide, when activated with light, provides a disinfectant that kills and/or inhibits the growth of microbes on the catheter surface. When photoactive titanium dioxide absorbs light with suitable photon energies, the titanium dioxide reacts with oxygen from the surrounding environment (air and/or, if present water). This reaction provides a self-cleaning and/or self-disinfecting effect. When a catheter includes photoactive titanium dioxide, the catheter will include self-cleaning and/or self-disinfecting characteristics.

Several organic molecules and microorganisms are destroyed or killed by oxidation. The antibacterial properties of a light activated titanium dioxide surface can be derived from the combination of two different characteristics of photoactive titanium dioxide containing materials, namely, self-cleaning and selfdisinfection characteristics. The self-cleaning characteristic provides anti-bacterial growth and/or anti-bacterial adhesion characteristics to the catheter surface. For example, degradation of organic substances by oxidation prevents bacteria and biofilm adhesion on the surface of the catheter. Furthermore, degradation of the organic material may kill or destroy the bacteria, thereby providing disinfection. When a surface containing titanium dioxide absorbs light (such as UV light, near UV light and/or visible light) the titanium dioxide reacts with surrounding oxygen to form hydroxyl (•OH) radicals. Hydroxyl free radicals are very powerful oxidizing agents that attack the structure of cell membranes and break down organic molecules. Thus, providing a self-cleaning and/or self-disinfecting catheter surface.

The titanium dioxide may be anatase, rutile, brookite or mixtures thereof. In solids, the outer electrons can potentially be found in two energetic bands, namely, the valence band and the conduction band. The energy gap between the valence band and the conduction band for titanium dioxide is in the region of 3.0-3.5 eV. With sufficient energy from exposure to light, electrons in titanium oxide can move from the valence band to the conduction band, thus initiating or activating a reaction with the titanium dioxide and surrounding oxygen.

Anatase shows a band gap of 3.2 e V, corresponding to a UV wavelength adsorption of 385 nm. In contrast, rutile has a smaller band gap of 3.0 eV, with excitation wavelengths that extend into the visible light range of 410 nm. In one embodiment, the catheters of the present disclosure may include a mixture of the different species of photoactive titanium dioxide. For example, the titanium dioxide could be a mixture of anatase-rutile or could be a mixture of brookiteanatase. In some instances the mixtures could be more active than the species is alone. In one embodiment, the photoactive titanium dioxide includes a mixture of anatase in an amount between 60% and 90% and rutile in an amount between 10% and 40%. In another alternative, the photoactive titanium dioxide includes a mixture of 80% anatase and 20% rutile. Furthermore, the catheter could also include one or more of zinc oxide, silver oxide and silicon dioxide. The catheter could include other additives as well. Additionally, the photoactive titanium dioxide may be in microparticle or nanoparticle form.

Photoactive titanium dioxide may be incorporated into the catheter and/or catheter surface in any suitable manner. For example, a portion of the catheter, such as the shaft and/or drainage member, may be made from a polymer that has been compounded (mixed or blended) with photoactive titanium dioxide. Polymer pellets may be mixed and melted with photoactive titanium dioxide to form a compounded polymer. The compounded polymer may then be extruded or molded to form the catheter or portions thereof.

Referring to Fig. 2 (not according to the claimed invention), the catheter shaft may be made by coextrusion. The coextruded catheter shaft may have an inner layer 22 and an outer layer 24. One or both of the inner layer 22 and the outer layer 24 may be made from a polymer compounded with photoactive titanium dioxide. In one embodiment, the outer layer 24 is made from a polymer compounded with photoactive titanium dioxide, while the inner layer 22 is made from a polymer with or without other additives.

Referring to Fig. 3, in this embodiment, the catheter 10 includes a substrate polymer that defines, for example, the catheter shaft 12 and/or the drainage member 20. In the embodiment illustrated in Fig. 3, a cross-section of the catheter shaft 12 is shown for illustrative purposes. The catheter shaft 12 includes a substrate 26 formed from a polymer. The polymer may be extruded or molded to form the substrate 26. Disposed over the surface of the substrate 26 is a coating 28 including photoactive titanium dioxide.

The coating includes a lubricious hydrophilic polymer, wherein the photoactive titanium dioxide is contained in the hydrophilic coating. The lubricious hydrophilic polymer is a cross-linked polymer. Furthermore, the lubricious hydrophilic polymer includes a matrix, wherein the photoactive titanium dioxide is contained within the matrix. The hydrophilic polymer may be any suitable hydrophilic polymer, such polyvinylpyrrolidone, polyethylene oxide, polyacrylic acid, polyacrylamide, hyaluronic acid, polyvinyl alcohol etc.

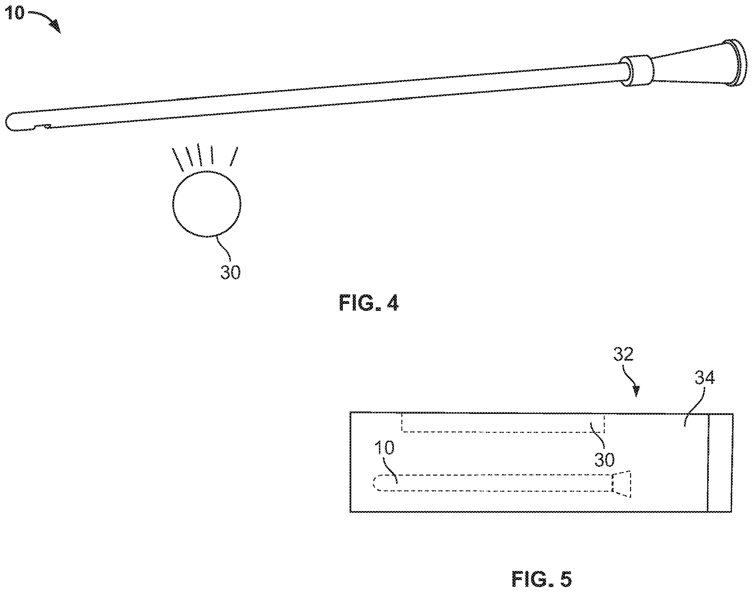

Referring now to Fig. 4, any of the reusable catheter products disclosed herein may include a light source 30 for exposing the catheter 10 to light. Depending on the particular application, the light source 30 may produce UV light, near UV light or visible light for activating the titanium dioxide. The light source may be, for example, one or more light emitting diodes. Furthermore, the light source may be powered by batteries, which may be rechargeable batteries. As discussed above, when the catheter is exposed to light, the photoactive titanium dioxide is activated to react with oxygen in the surrounding environment. The oxygen is oxygen from the surrounding liquid, water. The hydrophilic coating is activated to be lubricious by hydration with the hydration fluid, water. The hydration fluid may be present at the same time that the catheter is exposed to light. The light source 30 may be associated with an automatic shutoff switch or timer that shuts off the light after a selected time.

Referring to Fig. 5, there is shown one embodiment of a reusable catheter product 32. The product 32 includes a case 34 holding a catheter 10 and a light source 30. The catheter 10 may be any of the photoactive titanium dioxide catheters disclosed herein, and the light source 30 may be any of the light sources disclosed herein. In use, the user removes the catheter 10 from the case 34, and performs catheterization. The user then puts the catheter 10 back in the case 34 and activates the light source 30 to expose the catheter 10 to light, which activates the photoactive titanium dioxide. After the exposure to the light source, the catheter is ready for use. The case may also include a hydration fluid for hydrating the hydrophilic coating. The case may also include an indicator that indicates that the catheter has been exposed to a sufficient level of light or light energy. For example, the case may include a UV indication strip within the case that changes color upon exposure to selected amount of UV light. Such strips may be tuned to change color when exposed to a certain level of UV radiation.

## Claims

1. A reusable urinary catheter product, comprising:
an intermittent catheter comprising a catheter shaft, wherein the catheter shaft comprises polymer that defines a substrate;
a coating disposed on a surface of the substrate, the coating comprising a cross-linked lubricious hydrophilic polymer including a matrix; and
photoactive titanium dioxide contained within the matrix of the cross-linked lubricious hydrophilic polymer.

2. The product of claim 1, wherein the intermittent catheter further includes a drainage member comprised of polymer and photoactive titanium dioxide.

3. The product of any one of claims 1 and 2, wherein the polymer of the catheter shaft is compounded with photoactive titanium dioxide.

4. The product of claim 3, wherein the catheter shaft includes a coextruded tube including an inner polymer layer and an outer polymer layer, wherein the outer polymer layer comprises the polymer compounded with photoactive titanium dioxide.

5. The product of any one of claims 1-4, further including a light source that produces light that activates the photoactive titanium dioxide.

6. The product of claim 5, wherein the light source produces UV-light, near UV-light, visible light and/or mixtures thereof.

7. The product of any one of claims 5 and 6 further an automatic shut off switch that shuts off the light source after a selected period of time.

8. The product of any one of claims 5-7, further including a storage and disinfectant case containing the light source and the intermittent urinary catheter.

9. The product of claim 8, wherein the storage and disinfectant case further includes a hydration fluid comprising water for hydrating the lubricious hydrophilic coating.

10. The product of any one of claims 1-9, wherein the photoactive titanium dioxide comprises anatase, rutile, brookite, or mixtures thereof.

11. The product of any one of claims 1-10, wherein the photoactive titanium dioxide comprises a mixture of anatase in an amount between 90% and 60% and rutile in an amount between 10% and 40%.

12. The product of any one of claims 1-11, wherein the photoactive titanium dioxide comprises a mixture of 80% anatase and 20% rutile.

13. The product of any one of claims 1-12, wherein the catheter shaft further comprises one of more of zinc oxide, silver oxide and silicon dioxide.

## Patentansprüche

1. Wiederverwendbares Harnkatheterprodukt, umfassend:
einen intermittierenden Katheter, der einen Katheterschaft umfasst, wobei der Katheterschaft Polymer umfasst, das ein Substrat definiert;
eine Beschichtung, die auf einer Oberfläche des Substrats angeordnet ist, wobei die Beschichtung ein vernetztes, gleitfähiges, hydrophiles Polymer umfasst, das eine Matrix beinhaltet; und
in der Matrix des vernetzten, gleitfähigen hydrophilen Polymers enthaltenes photoaktives Titandioxid.

2. Produkt nach Anspruch 1, wobei der intermittierende Katheter ferner ein Dränageelement beinhaltet, das aus Polymer und photoaktivem Titandioxid besteht.

3. Produkt nach einem der Ansprüche 1 und 2, wobei das Polymer des Katheterschafts mit photoaktivem Titandioxid vermischt ist.

4. Produkt nach Anspruch 3, wobei der Katheterschaft einen koextrudierten Schlauch beinhaltet, der eine innere Polymerschicht und eine äußere Polymerschicht beinhaltet, wobei die äußere Polymerschicht das mit photoaktivem Titandioxid vermischte Polymer umfasst.

5. Produkt nach einem der Ansprüche 1-4, ferner beinhaltend eine Lichtquelle, die Licht erzeugt, das das photoaktive Titandioxid aktiviert.

6. Produkt nach Anspruch 5, wobei die Lichtquelle UV-Licht, Nah-UV-Licht, sichtbares Licht und/oder Mischungen davon erzeugt.

7. Produkt nach einem der Ansprüche 5 und 6, ferner einen automatischen Abschaltschalter, der die Lichtquelle nach einer ausgewählten Zeitspanne abschaltet.

8. Produkt nach einem der Ansprüche 5-7, ferner beinhaltend ein Lager- und Desinfektionsbehältnis, das die Lichtquelle und den intermittierenden Harnkatheter enthält.

9. Produkt nach Anspruch 8, wobei das Lager- und Desinfektionsbehältnis ferner ein Hydratationsfluid beinhaltet, das Wasser zum Hydratisieren der gleitfähigen hydrophilen Beschichtung umfasst.

10. Produkt nach einem der Ansprüche 1-9, wobei das photoaktive Titandioxid Anatas, Rutil, Brookit oder Mischungen davon umfasst.

11. Produkt nach einem der Ansprüche 1-10, wobei das photoaktive Titandioxid eine Mischung aus Anatas in einer Menge zwischen 90 % und 60 % und Rutil in einer Menge zwischen 10 % und 40 % umfasst.

12. Produkt nach einem der Ansprüche 1-11, wobei das photoaktive Titandioxid eine Mischung aus 80 % Anatas und 20 % Rutil umfasst.

13. Produkt nach einem der Ansprüche 1-12, wobei der Katheterschaft ferner Zinkoxid, Silberoxid oder Siliciumdioxid umfasst.

## Revendications

1. Produit de cathéter urinaire réutilisable, comprenant :
un cathéter intermittent comprenant un arbre de cathéter, dans lequel l'arbre de cathéter comprend un polymère définissant un substrat ;
un revêtement disposé sur une surface du substrat, le revêtement comprenant un polymère hydrophile lubrifiant réticulé comportant une matrice ; et
du dioxyde de titane photoactif contenu à l'intérieur de la matrice du polymère hydrophile lubrifiant réticulé.

2. Produit selon la revendication 1, dans lequel le cathéter intermittent comporte en outre un élément de drainage constitué de polymère et de dioxyde de titane photoactif.

3. Produit selon l'une quelconque des revendications 1 et 2, dans lequel le polymère de l'arbre de cathéter est mélangé à du dioxyde de titane photoactif.

4. Produit selon la revendication 3, dans lequel l'arbre de cathéter comporte un tube coextrudé comportant une couche de polymère interne et une couche de polymère externe, dans lequel la couche de polymère externe comprend le polymère mélangé à du dioxyde de titane photoactif.

5. Produit selon l'une quelconque des revendications 1 à 4, comportant en outre une source de lumière qui produit de la lumière qui active le dioxyde de titane photoactif.

6. Produit selon la revendication 5, dans lequel la source de lumière produit de la lumière UV, de la lumière proche UV, de la lumière visible et/ou des mélanges de celles-ci.

7. Produit selon l'une quelconque des revendications 5 et 6, en outre un commutateur d'arrêt automatique de la source de lumière après une période de temps sélectionnée.

8. Produit selon l'une quelconque des revendications 5 à 7, comportant en outre un étui de stockage et de désinfection contenant la source de lumière et le cathéter urinaire intermittent.

9. Produit selon la revendication 8, dans lequel l'étui de stockage et de désinfection comporte en outre un fluide d'hydratation comprenant de l'eau pour hydrater le revêtement hydrophile lubrifiant.

10. Produit selon l'une quelconque des revendications 1 à 9, dans lequel le dioxyde de titane photoactif comprend de l'anatase, du rutile, de la brookite ou des mélanges de ceuxci.

11. Produit selon l'une quelconque des revendications 1 à 10, dans lequel le dioxyde de titane photoactif comprend un mélange d'anatase en une quantité comprise entre 90 % et 60 % et de rutile en une quantité comprise entre 10 % et 40 %.

12. Produit selon l'une quelconque des revendications 1 à 11, dans lequel le dioxyde de titane photoactif comprend un mélange de 80 % d'anatase et de 20 % de rutile.

13. Produit selon l'une quelconque des revendications 1 à 12, dans lequel l'arbre de cathéter comprend en outre l'un ou plusieurs de l'oxyde de zinc, de l'oxyde d'argent et du dioxyde de silicium.
